Europäisches Patentamt

European Patent Office

Office européen des brevets

(11) Numéro de publication : **0 321 337 B1**

(12) **FASCICULE DE BREVET EUROPEEN**

(45) Date de publication du fascicule du brevet :
04.03.92 Bulletin 92/10

(51) Int. Cl.$^5$ : **A23K 1/00,** A23K 1/18,
A61K 9/52, A61K 9/22

(21) Numéro de dépôt : **88403174.1**

(22) Date de dépôt : **14.12.88**

(54) **Utilisation de compositions dégradables par voie enzymatique pour l'enrobage d'additifs alimentaires destinés aux ruminants.**

(30) Priorité : **15.12.87 FR 8717455**

(43) Date de publication de la demande :
**21.06.89 Bulletin 89/25**

(45) Mention de la délivrance du brevet :
**04.03.92 Bulletin 92/10**

(84) Etats contractants désignés :
**AT BE CH DE ES FR GB GR IT LI LU NL SE**

(56) Documents cités :
**EP-A- 0 188 953**
**EP-A- 0 268 533**
**US-A- 3 119 738**
**US-A- 4 066 754**
**JOURNAL OF ANIMAL SCIENCE, vol. 58, no. 1, 1984, pages 187-193, US; S.I. SMITH et al.: "Lipid coating as a mode of protecting free methionine from ruminal degradation"**

(73) Titulaire : **RHONE-POULENC SANTE**
**20, avenue Raymond Aron**
**F-92160 Antony (FR)**

(72) Inventeur : **Ardaillon, Pierre**
**10 Impasse Beau-Vallon**
**F-69800 Saint Priest (FR)**
Inventeur : **Bourrain, Paul**
**32/0 Chemin des Peupliers**
**F-69570 Dardilly (FR)**

(74) Mandataire : **Le Pennec, Magali et al**
**RHONE-POULENC SANTE, Service Brevets,**
**20 Avenue Raymond Aron**
**F-92165 Antony Cédex (FR)**

EP 0 321 337 B1

## Description

La présente invention concerne l'utilisation de compositions degradables par voie enzymatique pour l'enrobage d'additifs alimentaires destinés aux ruminants.

Les substances biologiquement actives administrées aux ruminants subissent généralement une dégradation au niveau du rumen et ne peuvent ainsi exercer pleinement leur activité lorsqu'elles progressent dans le tractus digestif. Il est donc nécessaire de protéger les substances biologiquement actives de telle manière qu'elles résistent à la dégradation dans le rumen.

Jusqu'à présent, il a été proposé d'enrober les substances biologiquement actives par des compositions que présentent la propriété d'être stables dans le rumen dont le pH est voisin de 6 et de permettre la libération de la substance active dans la caillette dont le pH est voisin de 2.

Généralement, les compositions d'enrobage connues sont constituées de l'association d'une substance sensible aux variations du pH, choisie en particulier parmi des copolymères basiques synthétiques, avec des substances hydrophobes qui peuvent être choisies, par exemple, parmi les acides gras ou leurs dérivés et les polymères hydrophobes et elles forment une pellicule continue autour de la substance biologiquement active. De telles compositions sont décrites, par exemple, dans les brevets français FR 78 23966 (2 401 620), FR 78 23968 (2 401 621) ou FR 81 18954 (2 514 261).

La sélection des compositions permettant la réalisation du but recherché est effectuée sur la base de leur différence de comportement en fonction du pH. Le but recherché est atteint lorsque la composition d'enrobage est stable à un pH supérieur ou égal à 5,5 pendant une longue période (24 à 48 heures) et lorsqu'elle est dégradée rapidement (quelques minutes à quelques heures) à un pH inférieur ou égal à 3,5. Il en résulte que des compositions d'enrobage dont la différence de comportement à pH=6 à pH=2 est faible ne peuvent pas, a priori, être utilisables dans le but recherché. Ainsi, dans la demande de brevet européen EP 0 188 953 sont revendiquées des compositions d'enrobage qui sont constituées d'un liant filmogène non hydrosoluble dont l'hydrophilie est contrôlée et d'une substance sensible aux variations du pH. Les exemples comparatifs qui sont donnés montrent que, en l'absence d'un des éléments des compositions revendiquées, on obtient des compositions qui sont a priori inutilisables dans le but recherché puisqu'elles ont des stabilités comparables à pH=6 et à pH=2.

Il a maintenant été trouvé, et c'est ce qui fait l'objet de la présente invention, qu'il est possible d'utiliser des composition d'enrobage stables en milieu basique et en milieu acide pour enrober des substances actives destinées aux ruminants qui sont libérées non pas au niveau de la caillette mais au niveau de l'intestin grêle tout en conservant leur activité bénéfique, la dégradation de l'enrobage étant effectuée par les enzymes présentes dans l'intestin.

Selon la présente invention, une substance biologiquement active peut être enrobée par une composition :
– qui est stable dans le rumen, ce qui implique une bonne résistance à la flore microbienne et aux enzymes de ce milieu,
– qui est relativement stable dans le caillette dont le pH est acide, et
– qui est fortement dégradée au niveau de l'intestin grêle grâce aux enzymes rencontrées entre la caillette et l'iléon.

Les compositions d'enrobage utilisables selon l'invention sont constituées :
– soit de zéine associée à un polymère non hydrosoluble choisi parmi les éthers ou les esters de cellulose non hydrosolubles tels que l'éthylcellulose, l'acétate de cellulose, le propionate de cellulose ou l'acétobutyrate de cellulose, et les esters polyvinyliques tels que l'acétate de polyvinyle, et éventuellement à un agent plastifiant tel que la triacétine ou le propylèneglycol, la zéine représentant généralement de 50 à 90 % en poids de la composition et l'agent plastifiant pouvant représenter jusqu'à 10 % en poids de la composition,
– soit de zéine associée à une substance hydrophobe dont le point de fusion est supérieur à 60°C choisie parmi les acides gras (acide stéarique, acide béhénique), les esters gras, les alcools gras et leurs mélanges, et éventuellement à un polymère non hydrosoluble choisi parmi les éthers ou les esters de cellulose non hydrosolubles tels que l'éthylcellulose, l'acétate de cellulose, le propionate de cellulose ou l'acétobutyrate de cellulose, et les esters polyvinyliques tels que l'acétate de polyvinyle, la zéine représentant généralement de 10 à 50 % en poids de la composition et le polymère non hydrosoluble pouvant représenter jusqu'à 10 % en poids de le composition.

Généralement, les compositions du premier type sont utilisées pour l'enrobage de substances peu solubles dans l'eau telles que le méthionine et celles du second type pour des substances hydrosolubles telles que le chlorhydrate de lysine.

Il est particulièrement important de remarquer que les compositions utilisables selon l'invention sont constituées de substances qui sont connues pour pouvoir être utilisées dans l'industrie alimentaire, ce qui apporte un avantage considérable sur les compositions antérieurement connues.

Les compositions selon l'invention son particulièrement utiles pour enrober des substances actives qui sont sensibles à la dégradation en milieu acide. De telles substances sont ainsi protégées tant à pH=6 (rumen) qu'à pH=2 (caillette) et elles ne sont libérées que dans l'instestin (pH=7) par suite de la destruction de la composition d'enrobage sous l'action des enzymes présentes dans l'intestin.

Les compositions d'enrobage utilisables selon l'invention peuvent être obtenues en dispersant ou en dissolvant la zéine dans une solution ou une dispersion du polymère non hydrosoluble et/ou de la substance hydrophobe, et éventuellement selon le cas l'agent plastifiant dans un solvant organique ou dans un mélange de solvants organiques convenables choisis en fonction de la nature spécifique des constituants. Généralement, la composition d'enrobage est obtenue après évaporation du ou des solvants.

Selon la présente invention, les compositions d'enrobage sont particulièrement utiles pour protéger des substances thérapeutiques ou nutritives diverses telles que des médicaments, des vitamines ou des acides aminés destinés à être administrés par voie orale à des ruminants. Les substances enrobées sont généralement mélangées à la nourriture des animaux.

Les substances enrobées sont généralement des granulés sous forme de microcapsules constituées d'un noyau central entouré d'une pellicule continue de la composition d'enrobage. Cependant, les substances actives peuvent être dispersées dans la composition d'enrobage. En général, la composition d'enrobage représente 5 à 60 % en poids du granulé ou de la dispersion.

Les granulés peuvent être obtenus par application des techniques connues. Selon la nature de la composition d'enrobage, on utilise soit des techniques d'extrusion ou de pulvérisation de solutions ou d'émulsions en lit fluidisé, soit des techniques d'encapsulation en milieu fondu ou semi-fondu, soit des techniques d'enrobage en milieu liquide telles que la coacervation.

Les granulés obtenus selon la présente invention sont stables au stockage et lors des manipulations, ne se détériorent pas lors de la confection des aliments pour animaux et ne sont pas détruits lors de leur absorption par les animaux et en particulier par écrasement ou par broyage lors de la mastication.

La taille des granulés sera fonction de l'utilisation qui en est faite et plus particulièrement elle sera déterminée en fonction de l'animal auquel ils sont destinés.

Il est possible d'enrober des substances actives pour obtenir des granulés dont la taille est comprise entre 0,1 et 5 mm.

D'un intérêt tout particulier sont les granulés qui contiennent comme substances actives la méthionine, la lysine ou des vitamines (vitamine A) dont le rôle est très important dans l'alimentation des animaux et plus spécialement des ruminants.

Les exemples suivants, donnés à titre non limitatif, illustrent la présente invention.

## EXEMPLE 1

Selon la technique d'encapsulation en spray-coating (décrite par exemple dans la demande de brevet européen EP 188 953) on prépare des granulés de méthionine enrobés par une composition d'enrobage constituée de zéine (69,2 %), d'éthylcellulose N22 (Herculès) (23 %) et de triacétine (7,7 %). Le titre en méthionine dans les granulés enrobés est de 72 %.

Par ailleurs, à titre de comparaison, on prépare des granulés de méthionine enrobés par les compositions suivantes :
- Granulés A :    Ethylcellulose N100 (Herculès) (80 %)
                  Copolymère styrène-vinyl-2 pyridine (30-70) (20 %)
Le titre en méthionine dans les granulés enrobés est de 70 %.
- Granulés B:    Ethylcellulose N100 (Herculès) (58,3 %)
                 Copolymère styrène-vinyl-2 pyridine (30-70) (25 %)
                 Propylèneglycol (16,7 %)
Le titre en méthionine dans les granulés enrobés est de 73 %.

Les différents granulés enrobés ainsi préparés sont soumis à des tests in vitro et in vivo.

## 1) Test in vitro

Le relargage de la méthionine contenue dans les granulés obtenus est examiné, dans des conditions déterminées, en agitant une quantité connue de granulés dans un milieu tamponné maintenu à pH constant à une température de 40°C. On compare les vitesses de relargage d'un échantillon soumis à différents pH.

Les résultats obtenus sont rassemblés dans le tableau 1.

## Tableau 1

| Produits | Titre en méthionine | % de méthionine libérée | | | | | | |
|---|---|---|---|---|---|---|---|---|
| | | à pH=6 après | | à pH=2 après | | | | |
| | | 6 heures | 24 heures | 1/2 h | 1 h | 2 h | 5 h | 7 h |
| Granulés selon l'exemple 1 | 72 | 9 | 19 | | | 5 | 12 | |
| Granulés A | 70 | 3 | 5,5 | 2 | | | 22 | |
| Granulés B | 73 | | | 1,3 | 2,5 | 3,6 | 6 | 8,5 |

### 2) Test in vivo

On détermine la résistance des granulés à un séjour dans le rumen, dans la caillette puis dans l'intestin grêle chez le mouton en mesurant la quantité d'azote totale disparue des sachets après chacune des trois étapes successives.

### 2.1 Digestion dans le rumen

Des sachets de nylon (maille carrée de 46 μm) contenant chacun environ 3 g de granulés enrobés, pesés exactement, sont incubés pendant 12 heures dans le rumen de 2 moutons à raison de 2 sachets par produit et par mouton.

Après incubation dans le rumen, les sachets sont lavés à l'eau froide puis lyophilisés pendant 48 heures. Les sachets sont pesés et la quantité de granulés ayant disparu est déterminée. Les résidus sont ensuite sortis des sachets et échantillonnés en double pour le dosage d'azote total.

Les résidus restant sont rassemblés par type de produit et sont placés dans de nouveaux sachets de nylon destinés à l'incubation dans la caillette.

### 2.2 Digestion dans la caillette

Trois sachets de nylon (maille carrée de 46 μm) dont la taille est sensiblement la moitié de celle des sachets utilisés pour la digestion dans le rumen contenant environ 0,5 g de résidu sont utilisés pour chaque durée d'incubation et pour chaque produit essayé.

Ces sachets sont introduits, attachés à une ficelle, dans la caillette de 3 moutons munis d'une canule de la caillette (diamètre intérieur 28 mm) et soumis au même régime alimentaire à raison de 1 sachet par mouton et par produit.

Après incubation pendant 0,5 heure, 2 heures et 4 heures, les sachets sont lavés, lyophilisés et pesés. Les résidus sont, d'une part, échantillonnés pour le dosage de l'azote et, d'autre part, utilisés pour l'étude de la digestion dans l'intestin grêle.

### 2.3 Digestion dans l'intestin grêle

Les résidus obtenus précédemment sont regroupés par temps d'incubation et par produit. Ils sont introduits dans des micro-sachets (disques de 20 mm de diamètre) de nylon (maille carrée de 46 μm) à raison de 200 mg de résidu par sachet et 3 sachets par temps d'incubation.

Ces sachets sont introduits à l'entrée de l'intestin grêle (duodénum) de trois moutons soumis au même régime alimentaire et munis d'une canule simple au niveau duodénal (5 cm de pylore, 12 mm de diamètre intérieur) et d'une canule réentrante (18 mm de diamètre intérieur) à la fin de l'intestin grêle (iléon terminal).

Les résidus de chaque type d'enrobant sont étudiés 2 par 2 au cours de 2 séries de mesure, chaque animal recevant 1 sachet de chacun des 6 traitements (2 enrobants x 3 durées d'incubation dans la caillette).

Les 6 sachets par animal sont introduits à intervalle de 15 minutes. Trois heures après le début de l'introduction, la canule réentrante est ouverte et le contenu iléal s'écoule librement à l'extérieur de l'animal. Les sachets sont expulsés après une durée de transit variant de 3,5 à 8 heures. Ils sont aussitôt recueillis et analysés comme précédemment.

Les résultats obtenus sont rassemblés dans le tableau 2.

**Tableau 2**

LIBERATION DES PRODUITS DANS DIFFERENTS COMPARTIMENTS DU TUBE DIGESTIF DU MOUTON

| | RUMEN | | | CAILLETTE | | | INTESTIN GRELE | |
|---|---|---|---|---|---|---|---|---|
| Méthionine | Temps h | Nombre d'essais | Bilan N total % disparu | Temps h | Nombre d'essais | Bilan N total % disparu | Nombre d'essais | Bilan N total % disparu |
| Produit selon l'exemple 1 | 12 | 4 | 6,64 ± 0,49 | 0,5 | 3 | 6,13 ± 0,06 | 2 | 95,70 ± 1,53 |
| | | | | 2 | 3 | 11,17 ± 1,23 | 3 | 96,57 ± 3,72 |
| | | | | 4 | 3 | 14,82 ± 0,13 | 3 | 95,72 ± 4,58 |
| Granulés A | 12 | 4 | 2,19 ± 0,68 | 0,5 | 3 | 0 | 3 | 7,06 ± 2,69 |
| | | | | 2 | 3 | 2,64 ± 0,19 | 3 | 6,66 ± 2,26 |
| | | | | 4 | 3 | 6,64 ± 0,57 | 2 | 2,51 ± 0,47 |
| Granulés B | 12 | 4 | 6,51 ± 0,51 | 0,5 | 3 | 1,37 ± 0,17 | 3 | 0 |
| | | | | 2 | 3 | 1,24 ± 0,11 | 3 | 0,58 ± 1,00 |
| | | | | 4 | 3 | 2,07 ± 0,07 | 3 | 0,69 ± 0,62 |

## EXEMPLE 2

Dans un mélange de 500 cm3 de dichlorométhane et de 500 cm3 d'éthanol absolu, on dissout 22 g de zéine et 88 g d'acide stéarique pur.

La solution obtenue est pulvérisée, par la technique du "spray-coating" sur 350 g de méthionine sous forme de granulés dont le diamètre moyen est compris entre 0,5 et 0,63 mm.

On obtient ainsi des granulés enrobés dont le titre en méthionine est de 74,5 %.

La quantité de méthionine libérée à partir des granulés après 24 heures à 40°C en milieu aqueux tamponné à pH=6 est de 6 % et à pH=2 est de 7 %.

Après un séjour de 48 heures dans le rumen de brebis, le taux de méthionine résiduelle est de 68,0 ± 6 %.

## EXEMPLE 3

Dans un mélange de 500 cm3 de dichlorométhane et de 500 cm3 d'éthanol absolu, on dissout 14 g de zéine, et 88 g d'acide stéarique pur et 8 g d'éthylcellulose N22.

La solution obtenue est pulvérisée, par la technique du "spray-coating" sur 350 g de chlorhydrate de lysine sous forme de granulés dont le diamètre moyen est voisin de 0,8mm.

On obtient ainsi des granulés enrobés dont le titre en chlorhydrate de lysine est de 69,4 %.

La quantité de chlorhydrate de lysine libéré à partir des granulés après 24 heures à 40°C en milieu tamponné à pH=6 est de 2,6 % et à pH=2 est de 6 %.

Après un séjour de 48 heures dans le rumen de brebis, le taux de chlorhydrate de lysine résiduel est de 78,0 ± 2 %.

**Revendications**

1. Utilisation d'une composition constituée soit de zéine associée à un polymère non hydrosoluble et éventuellement à un agent plastifiant, soit de zéine associée à une substance hydrophobe et éventuellement à un polymère non hydrosoluble pour l'enrobage d'additifs alimentaires afin d'obtenir des granulés, destinés à l'alimentation des ruminants, qui sont stables dans la panse et dans le caillette et qui sont dégradés par les enzymes de l'intestin en libérant l'additif alimentaire.

5

2. Utilisation selon la revendication 1 d'une composition constituée de zéine associée à un polymère non hydrosoluble choisi parmi les éthers ou les esters de cellulose non hydrosoluble et les esters polyvinyliques et éventuellement à un agent plastifiant tel que la triacétine ou le propylèneglycol, la zéine représentant 50 à 90 % en poids de la composition et l'agent plastifiant pouvant représenter jusqu'à 10 % en poids de la composition.

3. Utilisation selon la revendication 2 d'une composition dans laquelle le polymère non hydrosoluble est choisi parmi l'éthycellulose, l'acétate de cellulose, le propionate de cellulose, l'acétobutyrate de cellulose et l'acétate de polyvinyle.

4. Utilisation selon la revendication 1 d'une composition constituée de zéine associée à une substance hydrophobe dont le point de fusion est supérieur à 60°C et éventuellement à un polymère non hydrosoluble choisi parmi les éthers ou les esters de cellulose et les esters polyvinyliques, la zéine représentant de 10 à 50 % en poids de la composition et le polymère non hydrosoluble pouvant représenter jusqu'à 10 % en poids de la composition.

5. Utilisation selon la revendication 4 d'une composition dans laquelle la substance hydrophobe est choisie parmi les acides gras, les esters gras, les alcools gras et leurs mélanges.

6. Utilisation selon la revendication 4 d'une composition dans laquelle le polymère non hydrosoluble est choisi parmi l'éthylcellulose, l'acétate de cellulose, le propionate de cellulose, l'acétobutyrate de cellulose et l'acétate de polyvinyle.

7. Utilisation selon la revendication 1 pour l'enrobage d'additifs alimentaires choisis parmi les médicaments, les vitamines et les acides aminés.

8. Utilisation selon la revendication 1 pour l'enrobage d'additifs alimentaires choisis parmi la méthionine et la lysine.

## Patentansprüche

1. Verwendung einer Masse, bestehend entweder aus Zein in Kombination mit einem wasserunlöslichen Polymer und gegebenenfalls einem Weichmacher oder aus Zein in Kombination mit einer hydrophoben Substanz und gegebenenfalls einem wasserunlöslichen Polymer zur Umhüllung von Futtermittelzusätzen zwecks Herstellung von Granalien, die an Wiederkäuer verfüttert werden und im Pansen und im Labmagen stabil sind und die durch Verdauungsenzyme unter Freisetzung des Futtermittelzusatzes abgebaut werden.

2. Verwendung einer Masse nach Anspruch 1, bestehend aus Zein in Kombination mit einem wasserunlöslichen Polymer, ausgewählt aus den wasserunlöslichen Celluloseäthern oder -ersten und den Polyvinylestern, und gegebenenfalls einem Weichmacher wie Triacetin oder Propylenglykol, wobei das Zein 50 bis 90 Gew.-% der Masse ausmacht und der Weichmacher bis zu 10 Gew.-% der Masse ausmachen kann.

3. Verwendung einer Masse nach Anspruch 2, worin des wasserunlösliche Polymer ausgewählt ist aus Äthylcellulose, Celluloseacetat, Cellulosepropionat, Celluloseacetobutyrat und Polyvinylacetat.

4. Verwendung einer Masse nach Anspruch 1, bestehend aus Zein in Kombination mit einer hydrophoben Substanz, deren Schmelzpunkt über 60°C liegt, und gegebenenfalls einem wasserunlöslichen Polymer, ausgewählt aus den Celluloseäthern oder -estern und den Polyvinylestern, wobei das Zein 10 bis 50 Gew.-% der Masse ausmacht und das wasserunlösliche Polymer bis zu 10 Gew.-% der Masse ausmachen kann.

5. Verwendung einer Masse nach Anspruch 4, worin die hydrophobe Substanz ausgewählt ist aus den Fettsäuren, den Fettsäure-estern, den Fettalkoholen und ihren Mischungen.

6. Verwendung einer Masse nach Anspruch 4, worin das wasserunlösliche Polymer ausgewählt ist aus Äthylcellulose, Celluloseacetat, Cellulosepropionat, Celluloseacetobutyrat und Polyvinylacetat.

7. Verwendung nach Anspruch 1 zur Umhüllung von Futtermittelzusätzen, ausgewählt aus den Medikamenten, den Vitaminen und den Aminosäuren.

8. Verwendung nach Anspruch 1 zur Umhüllung von Futtermittelzusätzen, ausgewählt aus Methionin und Lysin.

## Claims

1. The use of a composition consisting either of zein in combination with a non-water-soluble polymer and optionally with a plasticizing agent, or of zein in combination with a hydrophobic substance and optionally with a non-water-soluble polymer, for the coating of feed additives in order to obtain granules, intended for the feeding of ruminants, which are stable in the rumen and in the abomasum and which are degraded by the enzymes of the intestine, releasing the feed additive.

2. The use according to claim 1 of a composition consisting of zein in combination with a non-water-soluble polymer, chosen from non-water-soluble cellulose ethers or esters and polyvinyl esters, and optionally with a plasticizing agent such as triacetin or propylene glycol, zein representing 50 to 90% by weight of the composition and the plasticizing agent being capable of representing up to 10% by weight of the composition.

3. The use according to claim 2 of a composition in which the non-water-soluble polymer is chosen from ethyl cellulose, cellulose acetate, cellulose propionate, cellulose acetate butyrate and polyvinyl acetate.

4. The use according to claim 1 of a composition consisting of zein in combination with a hydrophobic substance whose melting point is above 60°C, and optionally with a non-water-soluble polymer chosen from cellulose ethers or esters and polyvinyl esters, zein representing from 10 to 50% by weight of the composition and the non-water-soluble polymer being capable of representing up to 10% by weight of the composition.

5. The use according to claim 4 of a composition in which the hydrophobic substance is chosen from fatty acids, fatty esters, fatty alcohols and mixtures thereof.

6. The use according to claim 4 of a composition in which the non-water-soluble polymer is chosen from ethyl cellulose, cellulose acetate, cellulose propionate, cellulose acetate butyrate and polyvinyl acetate.

7. The use according to claim 1 for the coating of feed additives chosen from medicinal products, vitamins and amino acids.

8. The use according to claim 1 for the coating of feed additives chosen from methionine and lysine.